(19) Europäisches Patentamt
European Patent Office
Office européen des brevets

(11) Veröffentlichungsnummer: **0 002 033**
**B1**

(12) # EUROPÄISCHE PATENTSCHRIFT

(45) Veröffentlichungstag der Patentschrift: **30.12.81**

(21) Anmeldenummer: **78101335.4**

(22) Anmeldetag: **09.11.78**

(51) Int. Cl.³: **C 08 G 65/08,** C 07 C 141/08, C 07 C 139/00 // C07C39/16, C11D1/24

(54) **Oberflächenaktive oxalkylierte und gegebenenfalls sulfatierte Bisphenole und Verfahren zu ihrer Herstellung.**

(30) Priorität: **18.11.77 DE 2751519**

(43) Veröffentlichungstag der Anmeldung: **30.05.79 Patentblatt 79/11**

(45) Bekanntmachung des Hinweises auf die Patenterteilung: **30.12.81 Patentblatt 81/52**

(84) Benannte Vertragsstaaten: **BE CH DE FR GB NL**

(56) Entgegenhaltungen:
**DE-B-2310426**
**US-A-2174761**
**US-A-3395170**

(73) Patentinhaber: **BASF Aktiengesellschaft, Carl-Bosch-Strasse 38, D-6700 Ludwigshafen (DE)**

(72) Erfinder: **Oppenlaender, Knut, Dr., Otto-Dill-Strasse 23, D-6700 Ludwigshafen (DE)**
Erfinder: **Stork, Karl, Dr., In der Gewann 5, D-6840 Lampertheim (DE)**
Erfinder: **Daubach, Ewald, Dr., Thomas-Mann-Strasse 52, D-6700 Ludwigshafen (DE)**
Erfinder: **Herrmann, Manfred, Dr., Parkstrasse 23, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# Oberflächenaktive oxalkylierte und gegebenenfalls sulfatierte Bisphenole und Verfahren zu ihrer Herstellung

Die Erfindung betrifft neue oberflächenaktive Mittel, die sich in einigen Fällen als Dispergiermittel eignen.

Die neuen Verbindungen gehorchen der Formel I:

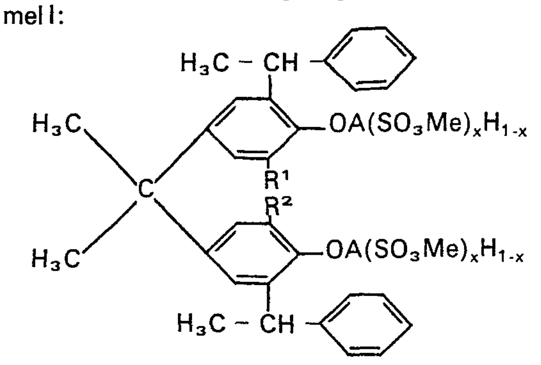

in der $R^1$ und $R^2$ gleich oder verschieden sind und für Wasserstoff oder den Rest

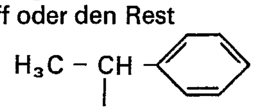

stehen, Me ein Alkali- oder gegebenenfalls substituiertes Ammoniumkation, x = 0 bis 1 und A den Rest eines Polymerisats des Äthylenoxids und/oder Propylenoxids mit insgesamt 5 bis 250 Alkylenoxidgruppen bedeuten.

Die Herstellung spielt sich in der Weise ab, dass man ein Phenol der Formel II:

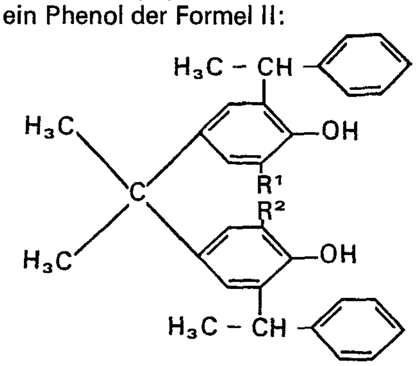

in der $R^1$ und $R^2$ gemäss Formel I definiert sind, mit Äthylenoxid und/oder Propylenoxid in an sich bekannter Weise in einer Menge umsetzt, dass pro phenolische Hydroxylgruppe 5 bis 250 Alkylenoxidgruppen gebunden sind, und das erhaltene Alkoxylierungsprodukt anschliessend pro freie Hydroxylgruppe mit x Mol eines Sulfatierungsmittels weiter umsetzt, wobei x gemäss Formel I definiert ist.

Die Herstellung der Ausgangsphenol erfolgt in an sich bekannter Weise durch Umsetzung von 2,2-(p,p'-Bishydroxydiphenyl)-propan (Bisphenol A) mit 2 bis 4 mol Styrol in Gegenwart eines sauren Katalysators. Man geht dabei zweckmässigerweise so vor, dass man unterschüssiges Styrol auf ca. 30 bis 70°C erhitzt und das Bisphenol zusammen mit einem Katalysator einträgt, wobei die Temperatur auf 100 bis 150°C ansteigt. Dann gibt man die restliche Menge des Styrols innerhalb 1½ bis 5 h zu und lässt noch einige Zeit nachreagieren.

Näturlich kann man den Ansatz auch auf einmal reagieren lassen, doch ist dann ein Arbeiten wegen der Schwierigkeiten der infolge der auftretenden Wärmetönung zu bewältigenden Wärmeabfuhr, vor allem bei grösseren Ansätzen nicht immer moglich.

Als saure Katalysatoren, die, bezogen auf eingesetztes Styrol, zu ca. 0,5 bis 1 Gew.% anwesend sind, verwendet man zweckmässigerweise z.B. Schwefelsäure, p-Toluolsulfonsäure, $AlCl_3$ oder andere Lewis-Säuren.

Das erhaltene Umsetzungsprodukt, das der Formel II entspricht, wird dann pro phenolischer OH-Gruppe mit soviel Äthylen- und/oder Propylenoxid umgesetzt, dass an jeder OH-Gruppe 5 bis 250 Alkylenoxidgruppen gebunden sind. Hierbei kann man in verschiedener Weise vorgehen.

Man kann ausschliesslich äthoxylieren und setzt dann — was im übrigen auch von der Bedeutung von $R^1$ und $R^2$ abhängig ist (Hydrophobierung durch die Phenyläthylreste!) — vorzugsweise mit 10 bis 250 mol Äthylenoxid pro OH-Gruppe um.

Man kann aber auch vorher Propylenoxid und anschliessend Äthylenoxid in einer Menge anlagern, dass die Gesamtsumme der gebundenen Alkylenoxidgruppen 250 nicht überschreitet. Hierbei sind bevorzugt bis zu 125 Propylenoxidgruppen gebunden.

Schliesslich kann man auch Propylenoxid allein oder Äthylenoxid und Propylenoxid nacheinander oder miteinander vermischt mit der Massgabe anlagern, dass die definitionsgemässe Gesamtzahl der Alkylenoxidgruppen eingehalten wird.

Bevorzugte Produkte sind die gemäss Anspruch 2 definierten Verbindungen, d.h. man lagert bevorzugt Propylenoxid und Äthylenoxid nacheinander oder Äthylenoxid allein an das mit 4 mol Styrol/mol Bisphenol A erhaltene Produkt an.

Die Alkoxylierung phenolischer Hydroxylgruppen ist eine altbekannte Reaktion und bedarf daher an sich keiner speziellen Erläuterung mehr.

Die Reaktion läuft in alkalischem Medium z.B. in Gegenwart von Alkalihydroxiden, wie NaOH oder KOH bei Temperaturen von 80 bis 150°C unter Drücken von 2 bis 10 bar ab und führt zu Verbindungen der Formel I, in der x = 0 ist.

Die Alkoxylierungsprodukte können dann gegebenenfalls mit einem Sulfatierungsmittel, vorzugsweise Chlorsulfonsäure oder Schwefeltrioxid, umgesetzt werden, wobei das Sulfatierungsmittel in einer so bemessenen Menge zum Einsatz gelangt, dass alle freien OH-Gruppen oder nur ein Teil davon sulfatiert wird. Im letzteren Fall entstehen Gemische aus Verbindungen der Formel I, die freie und sulfatierte Hydroxylgruppen enthalten.

Zum Schluss werden die entstandenen Schwefelsäurehalbester neutralisiert. Hierfür verwendet man in üblicher Weise Alkalihydroxide wie NaOH oder KOH, Ammoniak oder Amine, wie Triäthyl- oder Triisopropylamin oder Mono-, Di- oder Triäthanol- (oder -isopropanol)amine.

Sowohl die nichtsulfatierten als auch die neutralisierten sulfatierten Verbindungen der Formel I sind ausgezeichnete oberflächenaktive Mittel, von denen einige, insbesondere die nach Anspruch 2 definierten, eine hervorragende Dispergierwirkung für alle möglichen Stoffe zeigen — eine spezielle Verwendung wird in unserer Parallelanmeldung Nr. DE-A 2745449 detailliert beschrieben.

Die nun folgenden Beispiele erläutern die Erfindung. Teile sind Gewichtsteile, wenn sie nicht anders bezeichnet werden. Hierbei verhalten sich Gewichtsteile zu Raumteilen wie Kilogramm zu Liter.

*Beispiele*

a) Herstellung des Phenolderivates der Formel II

312 Teile (≅ 3 Molteile) Styrol werden in einem 4000 Raumteile fassenden Rundkolben mit Rührer, Tropftrichter, Thermometer und Rückflusskühler vorgelegt und auf 50° C erwärmt. Bei dieser Temperatur werden 684 Teile (≅ 3 Molteile) 2,2-(p,p'-Bishydroxydiphenyl)propan eingetragen und zu der Suspension 9,7 Teile wasserfreie p-Toluolsulfonsäure gegeben. Nach kurzer Zeit tritt eine exotherme Reaktion ein, wobei sich die Reaktionslösung auf etwa 120 bis 140° C erwärmt. Es resultiert ein klares Öl. Zu dieser Reaktionsmischung werden innerhalb von 3 h bei einer Temperatur von 120 bis 140° C weitere 936 Teile (≅ 9 Molteile) Styrol zugegeben. Zur Vervollständigung der Reaktion wird noch 1 h bei 120 bis 140° C nachgerührt. Das Produkt, das bei Raumtemperatur sehr viskos ist, wird bei 70 bis 90° C aus dem Rundkolben entfernt.

Farbe des Produktes: rotbraun

Ausbeute: 1896 g

b) Oxalkylierung

b1) In einem Autoklaven werden 1896 Teile (= 3 Molteile) des nach a) erhaltenen Umsetzungsproduktes und 19 g Kaliumhydroxidpulver vorgelegt und unter Rühren bei 120° C 870 Teile (= 15 Molteile) Propylenoxid in Portionen aufgepresst, wobei der Druck nicht über 3 bar steigt.

Dann werden bei der gleichen Temperatur 13200 Teile (300 Molteile) Äthylenoxid in gleicher Weise aufgepresst.

Das Umsetzungsprodukt wird warm aus dem Autoklaven abgelassen und erstarrt nach dem Abkühlen zu einer farblosen Masse.

Schmelzpunkt: ca. 52° C.

Für den Fall x = 0 in Formel I wird das nach a) erhaltene Umsetzungsprodukt nur mit Äthylenoxid bei 120° C unter den gleichen Bedingungen umgesetzt:

b2) 1896 Teile (= 3 Molteile) des nach a) hergestellten Produktes werden im Autoklaven mit 19 g Kaliumhydroxidpulver gemischt und unter Rühren bei 120° C 13200 Teile (= 300 Molteile) Äthylenoxid in Portionen aufgepresst, dass der Druck nicht über 3 bar steigt.

Das Oxäthylierungsprodukt (15115 Teile) wird noch warm abgelassen und erstarrt zu einer farblosen Masse.

Schmelzpunkt: ca. 50° C.

c) Herstellung der Schwefelsäurehalbester

15115 Teile (= 3 Molteile) des nach b2) erhaltenen Äthylenoxidadduktes werden aufgeschmolzen und in die auf 50 bis 60° C abgekühlte Schmelze bei dieser Temperatur 349,5 g Chlorsulfonsäure innerhalb von 10 min. eingetropft. Zur Vervollständigung der Umsetzung wird 30 min. bei 50 bis 70° C nachgerührt.

Dann wird das Reaktionsgemisch mit ca. 50%iger wässriger Natronlauge bei Temperaturen unterhalb von 70° C neutralisiert (pH-Wert: 6 bis 7). Das Produkt kann bei 60 bis 70° C ausgegossen werden und erstarrt beim Abkühlen.

Ausbeute: 15428 Teile, gerechnet als oberflächenaktives Mittel.

In gleicher Weise kann die Umsetzung auch mit Schwefeltrioxid durchgeführt werden.

Analog wurden weitere wasserlösliche oberflächenaktive Mittel (b) und (c) hergestellt. Diese sind in der folgenden Tabelle zusammengestellt.

Die Mittel sind durch das zugrunde liegende Phenol, die je mol Phenol angelagerte Menge Propylenoxid und Äthylenoxid sowie durch die je Mol Addukt angewandte Menge Chlorsulfonsäure charakterisiert.

*Tabelle*

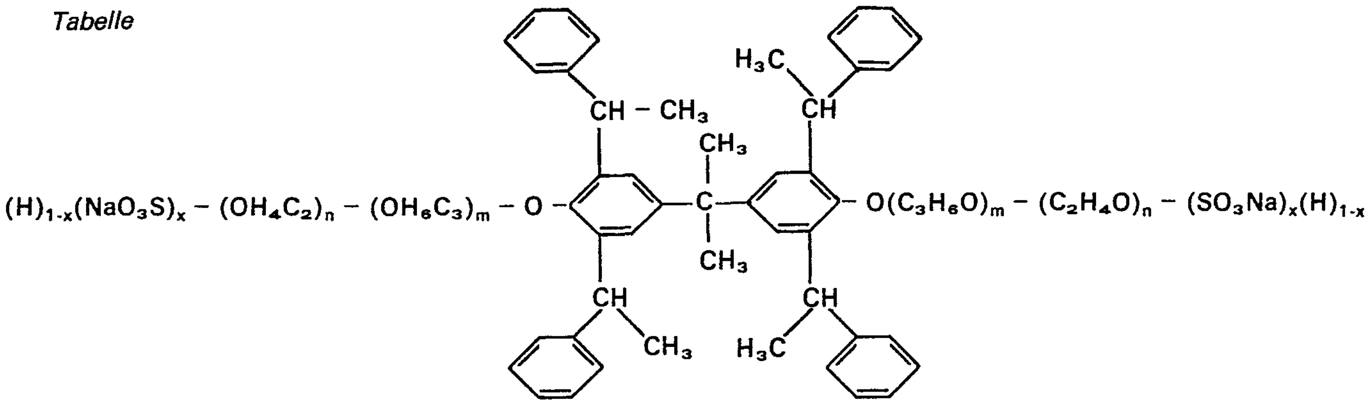

| Wasserlösliches oberflächenaktives Mittel Beispiel Nr. | Angelagerte Menge | | Mol Chlorsulfonsäure/ ½ Mol Addukt: $\overline{x}$ | Schmelzpunkt (°C) |
|---|---|---|---|---|
| | Mol Propylenoxid/ ½ Mol Phenol: $\overline{m}$ | Mol Äthylenoxid/ Phenol: $\overline{n}$ | | |
| 1 | 0 | 37,5 | 0 | 48 |
| 2 | 0 | 50 | 0 | 50 |
| 3 | 0 | 62,5 | 0 | 52 |
| 4 | 0 | 75 | 0 | 54 |
| 5 | 0 | 100 | 0 | 56 |
| 6 | 0 | 150 | 0 | 58 |
| 7 | 0 | 250 | 0 | 60 |
| 8 | 2,5 | 50 | 0 | 52 |
| 9 | 50 | 75 | 0 | viskos |
| 10 | 50 | 100 | 0 | viskos |
| 11 | 100 | 125 | 0 | Öl |
| 12 | 0 | 37,5 | 1,0 | 48 |
| 13 | 0 | 50 | 0,5 | 50 |
| 14 | 0 | 100 | 0,5 | 57 |
| 15 | 0 | 62,5 | 1,0 | 52 |
| 16 | 0 | 150 | 0,5 | 58 |
| 17 | 0 | 250 | 0,5 | 60 |
| 18 | 2,5 | 75 | 1,0 | 54 |
| 19 | 50 | 50 | 1,0 | Öl |
| 20 | 50 | 75 | 1,0 | Öl |
| 21 | 50 | 100 | 1,0 | viskos |
| 22 | 100 | 125 | 1,0 | viskos |
| 23 | 0,5 | 50,0 | 0,5 | 52 |
| 24 | 1,0 | 50 | 0,5 | 52 |

**Patentansprüche**

1. Verbindung der Formel I:

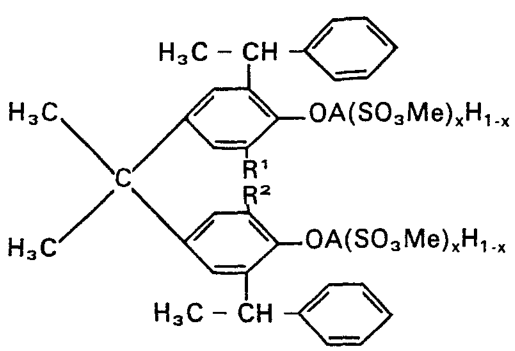

in der $R^1$ und $R^2$ gleich oder verschieden sind und für Wasserstoff oder den Rest

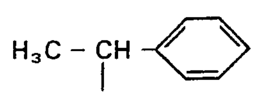

stehen, Me ein Alkali- oder gegebenenfalls substituiertes Ammoniumkation, x = 0 bis 1 und A den Rest eines Polymerisats des Äthylenoxids und/oder Propylenoxids mit insgesamt 5 bis 250 Alkylenoxidgruppen bedeuten.

2. Verbindung der Formel I gemäss Anspruch 1, in der $R^1$ und $R^2$ für den Rest

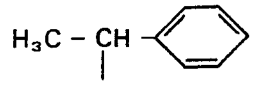

stehen.

3. Verfahren zur Herstellung von Verbindungen der Formel I gemäss den Ansprüchen 1 und 2, dadurch gekennzeichnet, dass man ein Phenol der Formel II

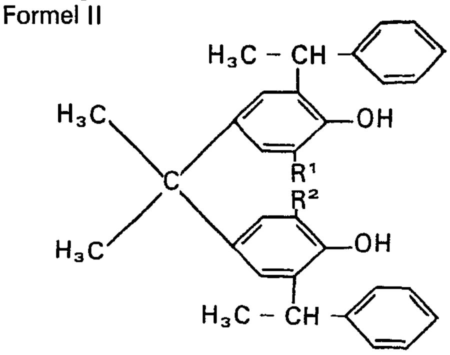

in der $R^1$ und $R^2$ gemäss Formel I definiert sind, mit Äthylenoxid und/oder Propylenoxid in an sich bekannter Weise in einer Menge umsetzt, dass pro phenolische Hydroxylgruppe 5 bis 250 Alkylenoxidgruppen gebunden sind, und das erhaltene Alkoxylierungsprodukt anschliessend pro freie Hydroxylgruppe mit x mol eines Sulfatierungsmittels weiter umsetzt, wobei x gemäss Formel I definiert ist.

## Claims

1. A compound of the formula I

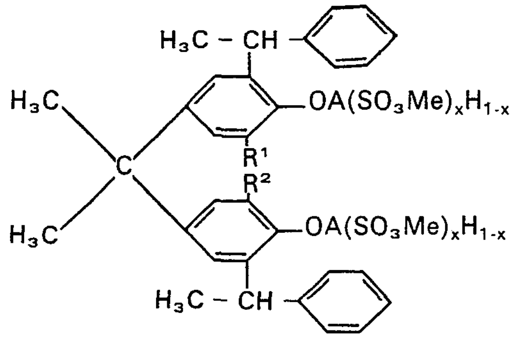

where $R^1$ and $R^2$ are identical or different and each is hydrogen or

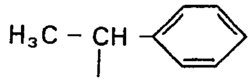 ,

Me is an alkali metal cation or a substituted or unsubstituted ammonium cation, x is from 0 to 1 and A is a radical of a polymer of ethylene oxide or of propylene oxide, the radical containing a total of from 5 to 250 alkylene oxide units.

2. A compound of the formula I as claimed in claim 1, where $R^1$ and $R^2$ are each

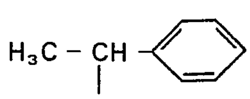 .

3. A process for the preparation of a compound of the formula I as claimed in claims 1 and 2, characterized in that a phenol of the formula II

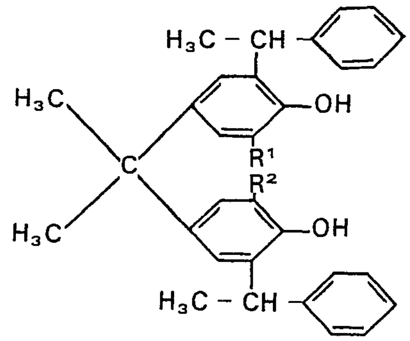

where $R^1$ and $R^2$ are defined as in formula I, is reacted with ethylene oxide, propylene oxide or a mixture of these in the conventional manner, using such amounts that from 5 to 250 alkylene oxide units are bonded per phenolic hydroxyl, and the resulting oxylalkylation product is then reacted further with x mol of a sulfating agent per free hydroxyl group, x being as defined in formula I.

## Revendications

1. Composé de la formule I

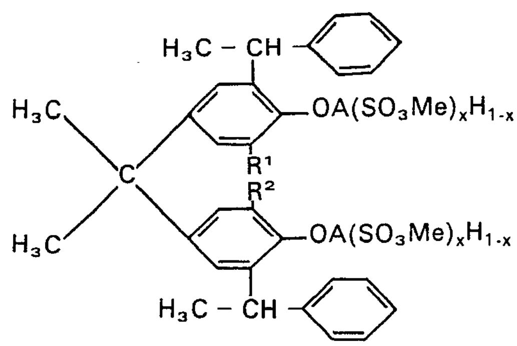

dans laquelle $R^1$ et $R^2$, qui peuvent être identiques ou différents, désignent soit un atome d'hydrogène, soit un reste

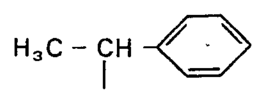 ,

Me représente un cation de métal alcalin ou un cation ammonium éventuellement substitué, x vaut 0 à 1 et A représente le reste d'un polymérisat de l'oxyde d'éthylène et/ou de l'oxyde de propylène avec un total de 5 à 250 groupes oxyde d'alcoylène.

2. Composé de la formule I suivant la revendication 1, dans laquelle $R^1$ = $R^2$ =

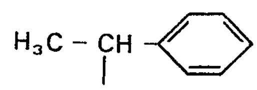 .

3. Procédé de préparation de composés de la formule I suivant les revendications 1 et 2, caractérisé en ce que l'on fait réagir, de manière connue en soi, un phénol de la formule II

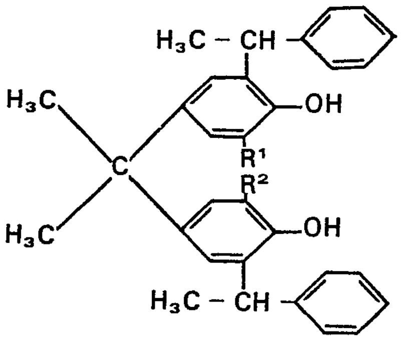

dans laquelle $R^1$ et $R^2$ possèdent la signification définie pour la formule I, avec de l'oxyde d'éthylène et/ou de l'oxyde de propylène en des proportions telles qu'il y a fixation de 5 à 250 groupes oxyde d'alcoylène par groupe oxhydryle phénolique, puis le produit d'oxalcoylation obtenu avec x mol d'un agent de sulfatation par groupe oxyhydryle libre, x ayant la signification définie pour la formule I.